# EUROPEAN PATENT SPECIFICATION

(11) **EP 3 808 342 B1**
(45) Date of publication and mention of the grant of the patent: **04.12.2024**
(21) Application number: 19202932.0
(22) Date of filing: 14.10.2019
(51) Int. Cl.: A61K 9/20, A61K 36/68, A61K 36/899, A61K 31/198, A61K 31/355, A61K 33/06

(54) **NATURAL AND/OR ORGANIC DISINTEGRANT PRE-MIXES FOR (ORAL) SOLID DOSAGE FORMS**
NATÜRLICHE UND/ODER BIO-ZERTIFIZIERTE SPRENGMITTEL-VORMISCHUNGEN FÜR (ORALE) FESTE DARREICHUNGSFORMEN
PRÉ-MÉLANGES DE DÉSINTÉGRANTS NATURELS ET/OU ORGANIQUES POUR DES FORMES POSOLOGIQUES SOLIDES (ORALES)

(43) Date of publication of application: 21.04.2021
(73) Proprietor: Bonutra AG, 6312 Steinhausen (CH); Herbitat Lifesciences, LLP, Mumbai 400055 (IN)
(72) Inventor: Patel,, Ritesh, Somerset, NJ New Jersey 08873 (US); Ghatak,, Somsuvra, Somerset, NJ New Jersey 08873 (US); Specht, Dr., Felix, Shelbyville, KY Kentucky 40065 (US); Yunis, Dr., Mahmud, 65203 Wiesbaden (DE)
(74) Representative: Uexküll & Stolberg

(56) References cited:
- WO-A1-00/74501
- GB-A- 2 201 875
- US-A- 5 227 248
- US-A- 5 445 826
- US-A1- 2009 197 974
- US-A1- 2017 281 584
- MD TAUSIF ALAM ET AL: "FDA-Approved Natural Polymers for Fast Dissolving Tablets", JOURNAL OF PHARMACEUTICS, vol. 2014, 27 April 2014 (2014-04-27), pages 1 - 6, XP055679291, ISSN: 2090-9918, DOI: 10.1155/2014/952970

## Description

### BACKGROUND OF THE INVENTION

### FIELD OF THE INVENTION

The invention relates to the field of natural disintegrant pre-mixes which are useful for formulating solid dosage forms for oral administration of drugs, vitamins, food supplements, etc. The pre-mixes promote the break-up of the tablet (and capsule "slugs") into smaller fragments in an aqueous environment thereby increasing the available surface area and promoting the release of the active nutraceutical/pharmaceutical substance.

### DESCRIPTION OF RELATED ART

In past years, the consumer awareness in health and natural foods has increased and led to a movement denominated "*Clean Label*"*.* The Clean Label movement avoids using artificial products labeled with E-Numbers, returning to natural foodstuff, paying attention to the packaging and the ingredients in the food. Consequently, the utilization of natural ingredients in the formulation of food and pharmaceutical products has progressively increased. In response to the consumer preference, many products with natural ingredients are being established which has resulted in increasing the market share of these naturally constituted products. In recent years, there has been a growth in natural product developments for a variety of purposes.

Nevertheless, the efforts in formulating all-natural or substantially all-natural nutritional supplements in a solid dosage form contained certified organic natural ingredients have generated intolerable outcomes. In particular, these developments have not lead to reliable characteristics like acceptable tablet hardness, good friability and/or acceptable dissolution profile of the active ingredient. Moreover, the use of natural ingredients so far resulted in adverse processing characteristics, such as reduced flowability, poor compressibility of the powder blend and batch to batch variation of finished product.

In the pharmaceutical and food industries, a number of excipients are used in the manufacture of solid dosage forms. These excipients are widely responsible for the physical characteristics or processing performance of solid dosage forms. The excipients in solid dosage formulations are generally inert substances which deliver the desired dosage and precise administration of active pharmaceutical ingredients. The solid dosage forms known up to date provide multi-functional roles such as improving the patient acceptability and enhancing the bioavailability and/or solubility of the active pharmaceutical ingredients.

Tablets are the most accepted dosage form among pharmaceutical and/or nutritional products. Tablets are manufactured by compressing a suitable powder mixture in a die at high compression force. The powder mixture contains, besides the active pharmaceutical ingredients, usually also fillers, binders, lubricants, glidants and disintegrants. The industrial production of high quality tablets involves a tablet mixture with good properties regarding homogeneity, flowability and compressibility.

Document US5445826A discloses a tablet comprising 37.19%wt guar gum, as a disintegrant, 4.13%wt oat fiber and 12.40%wt microcrystalline cellulose.

There are two main methods to manufacture tablets: **Direct compression:** the powder mixture is mixed during a period of time and is then directly compressed into tablets. This method is seen as the most efficient and desirable method. The choice of disintegrants is extremely critical for disintegration of direct compression tablets.

**Granulation techniques:** consist of wet granulation and dry granulation/slugging methods where binding agents are added in liquid form and in dry form respectively. Some actives exhibit poor fluidity and compressibility and in those case granules with acceptable flow and compression properties have to be formed.

A disintegrant is an excipient in a tablet formulation which is added to the formulation to promote the tablet's disintegration. It assists to break down the tablet into smaller particles such that the surface area is increased when the tablet comes into contact with liquid or fluid matter. In this way, the release of the active nutraceutical and/ir pharmaceutical substance is promoted. The function of disintegrant is opposite to the function of the binder. Strong binders thus require strong disintegrants to break down the tablet and release the active nutraceutical and/or pharmaceutical substance.

Super disintegrants are being developed due to the market demands for even faster absorption of the active nutraceutical and/or pharmaceutical substance. Super disintegrants assist in greater effectiveness of the active nutraceutical and/or pharmaceutical substance in lower dosage.

Natural polymers are safe, nontoxic and biocompatible. Therefore, there is a strong demand to use natural polymers as super disintegrant.

To the knowledge of the inventors, up to now there is no described or commercially available ready-to-use natural disintegrant pre-mix in powder form, which exclusively consists of natural polymers, which is suitable for manufacturing tablets, and which shows the properties of a super disintegrant.

Therefore, the object of the present invention is providing ready-to-use natural disintegrant pre-mix in powder form, which exclusively consists of natural polymers, which is suitable for manufacturing tablets, and which shows the properties of a super disintegrant.

Another object of the present invention is to provide an all-natural super disintegrant compound which is prepared by a physical blending procedure without carrying out any chemical reaction process.

### SUMMARY OF THE INVENTION

In one embodiment the present invention relates to a natural disintegrant composition in powder form exclusively consisting of natural polymers which comprises from about 30% to about 90% by weight of Psyllium Husk and about 20% to about 40% by weight of Oat Fiber, about 1% to about 20% by weight of at least one further component selected from Apple Fiber, Wheat Fiber, Pea Fiber, Cellulose Powder, Agave Powder, Bamboo Fiber, and a combination thereof (all weight percentages being based on the dry weight of the disintegrant composition).

**Psyllium Husk:** Psyllium is a soluble fiber obtained from the seeds of Plantago ovata. It is usually found in the form of husk, granules, capsules or powder. The plant contains mucilage in the epidermis of the seeds and has very high percentage of swelling index as compared to other super disintegrating agents. The rapid disintegration is due to the swelling of super disintegrants to create enough hydrodynamic pressure for quick and complete disintegration of the tablet (and capsule "slugs'). The rate of swelling and the significant force of swelling also determine its disintegrating efficiency.

**Oat Fiber:** Oat Fiber is typically an insoluble fiber made by grinding and purifying the outermost protective layer of the oat grain, composed of lignin, cellulose, and hemicellulose. It is a rich source of insoluble dietary fiber (up to 90%). FDA confirmed oat fiber as dietary fiber under mixed plant cell wall fibers category.

**Guar Gum:** also called guaran, is a substance made from guar beans, which has thickening and stabilizing properties. The guar seeds are dehusked, milled and screened to obtain the guar gum. It is typically produced as a free-flowing, off-white powder. It is classed as a galactomannan. In Europe, guar gum has EU food additive code E412. The disintegration of the tablet might be due to its instantaneous swelling characteristics when it comes into contact with water and owing to its high hydrophilic nature.

**Other natural gum components:** Locust bean gum, gum karaya, Aegle marmelose gum, Mangifera indica gum and similar other plant derived gums and mixtures of the foregoing.

**Natural Plant Fibers/Dietary Fibers:** Apple Fiber, Wheat Fiber, Pea Fiber, Cellulose Powder, Agave Powder, Bamboo Fiber and similar other plant/dietary fibers and mixtures of the foregoing.

Another objective of the present invention is to provide a method for manufacturing an all-natural super disintegrant composition as specified in claim 5. In an embodiment, the present invention relates to a method for preparing a disintegrant composition according to any of the previous embodiments, wherein the ingredients are mixed by physical blending them without carrying out any chemical reaction process.

### DETAILED DESCRIPTION OF THE INVENTION

In an embodiment of the invention, the natural disintegrant composition pre-mix according to any of the embodiments described herein-above is used in the manufacture of solid dosage forms for oral administration of drugs, vitamins, food supplements. The manufacture of solid dosage forms can be accomplished either by direct compression or granulation techniques.

It has been found by the inventors that the natural disintegrant composition pre-mix according to any of the embodiments described herein-above enhances the disintegration properties of the tablets, if used in direct compression or in granulation procedures. In fact, the natural disintegrant composition provides super disintegrant properties to the tablet, while maintaining excellent properties of homogeneity, flowability and compressibility during the manufacture of the solid oral dosage form.

The content of natural disintegrant composition pre-mix according to any of the embodiments described herein-above in the tablet may be about 1 to 10% w/w, preferably between 3 to 5% w/w, based on the total weight of the tablet. Natural excipients are preferably used in the pharmaceutical and food industry because of their biocompatibility, non-toxicity, low cost and renewable resources for a sustainable supply. Furthermore, the tablet formulated with an all-natural disintegrant composition pre-mix has a great acceptability by the consumers, which is due to utilization of clean labeled ingredients.

The quantity of the Psyllium Husk, which is the main disintegrant in the premix, is within the range about 30% to about 90% by weight of the dry disintegrant composition. The grade of the Psyllium Husk can be organic and/or according to EP and USP/NF. A range of about 30% to 70% by weight of the dry disintegrant composition is preferred.

The quantity of secondary disintegrant, Oat Fiber, in the premix is within the range of about 20% bout 40% by weight of the dry disintegrant composition. The grade of the Oat Fiber can be organic certified. A range of 20% to 40% by weight of the dry disintegrant composition is preferred.

A plant fiber/dietary fiber like Bamboo Fiber or Agave Powder can be added into the disintegrant composition within a range of 1% to about 20% by weight of the dry disintegrant composition. The plant fiber/dietary fiber improves the manufacturing of the pre-mix. The grade of the fiber can be organic certified or conventional. A range of 1% to 8% by weight of the dry disintegrant composition is preferred.

A gum component such as guar gum can alternatively or additionally be added to the premix within the range of 1% to about 25% by weight of the dry disintegrant composition. The grade of guar gum can be organic certified or conventional. A range of 1% to 15% by weight of the dry disintegrant composition is preferred.

Hereinafter, the present invention will now be described in detail with reference to the appending examples. The scope of the present invention is limited to the scope of the appended claims.

### EXAMPLES

The following examples 1 to 5 illustrate natural disintegrant compositions according to present invention. All percentages are by weight.

### Example 1:

A dry premix composition is produced by simple mixing of the ingredients as set out in Table 1 in a dry powder mill/blender, such as a V-type powder blender until a homogenous mixture is achieved, resulting in a formulation of the inventive disintegrant premix composition for manufacturing solid dosage forms. Optionally the individual ingredient or the dry powder blend may also be milled to form a fine powder to enhance the functionality and performance of the super disintegrant blend in solid oral dosage forms such as tablets (and capsule 'slugs').

**Table 1:**

| **Excipient:** | **Grades:** | **% w/w** |
|---|---|---|
| Psyllium Husk | USP / NF /organic/Conventional | 60 |
| Oat Fiber | Organic/Conventional | 37 |
| Bamboo Fiber | Organic/Conventional | 1.9 |
| Agave Powder | Organic/Conventional | 1.1 |

### Examples 2 to 5

In the following examples, the dry disintegrant premix compositions were produced as in Example 1, however with different ingredients:

**Example 2 (Table 2):**

| **Excipient:** | **Grades:** | **% w/w** |
|---|---|---|
| Psyllium Husk | USP / NF /organic/Conventional | 50 |
| Oat Fiber | Organic/Conventional | 30 |
| Guar Gum | Organic/Conventional | 15 |
| Agave Powder | Organic/Conventional | 5 |

**Example 3 (Table 3):**

| **Excipient:** | **Grades:** | **% w/w** |
|---|---|---|
| Psyllium Husk | USP / NF /organic/Conventional | 60 |
| Oat Fiber | Organic/Conventional | 35 |
| Bamboo Fiber | Organic/Conventional | 1.9 |
| Pea Fiber | Organic/Conventional | 2.1 |

**Example 4 (Table 4):**

| **Excipient:** | **Grades:** | **% w/w** |
|---|---|---|
| Psyllium Husk | USP / NF /organic/Conventional | 60 |
| Oat Fiber | Organic/Conventional | 30 |
| Bamboo Fiber | Organic/Conventional | 5 |
| Wheat Fiber | Organic/Conventional | 5 |

**Example 5 (Table 5):**

| **Excipient:** | **Grades:** | **% w/w** |
|---|---|---|
| Psyllium Husk | USP / NF /organic/Conventional | 60 |
| Oat Fiber | Organic/Conventional | 30 |
| Bamboo Fiber | Organic/Conventional | 5 |
| Apple Fiber | Organic/Conventional | 5 |

### RESULTS AND DISCUSSION ON THE INFLUENCE OF THE INVENTION ON TABLET DISINTEGRATION

### Example 6

The present invention was developed to ensure efficient tablet disintegration and batch to batch consistency.

According to this, a test series was defined to validate the influence of the invention on tablet disintegration time. Therefore, several formulations with different disintegrant contents and different active nutraceutical/pharmaceutical substances (Calcium, Vitamin E, L-Arginine) were investigated due to their disintegration time.

Consequently, tablets were compressed with a standard round tooling with 10 mm diameter, 5 mm thickness and engraving. For the characterization, the tablets were tested on disintegration time (according to Ph. Eur. 2.9.1).

The preferred formulas for the testing of super disintegrant premix composition are set out in the following table:

**Table 6:**

| **Excipient** | **Grades** | **Formulation I (% w/w)** | **Formulation II (% w/w)** |
|---|---|---|---|
| Psyllium Husk | USP / NF /organic | 60 | 50 |
| Oat Fiber | organic | 37 | 30 |
| Guar Gum | organic | N/A | 15 |
| Bamboo Fiber | organic | 1.9 | N/A |
| Agave Powder | organic | 1.1 | 5 |

The following table illustrates the used formulations and the results. All percentages are by weight. Croscarmellose were used as reference super disintegrant.

| | | | **disintegration time** | | | | | | |
|---|---|---|---|---|---|---|---|---|---|
| | | | no **disintegrants** | **+ 3 % crosscarm.** | **+ 5 % crosscarm.** | **+ 5 % formulation 1** | **+ 3 % formulation 1** | **+ 5 % formulation 2** | **+ 3 % formulation 2** |
| **tablet formula** | **%** | | | | | | | | |
| MCC 200 | 65,55 | | 28:13 min | 6:21 min | 4:07 min | 1:30 min | 1:43 min | 1:50 min | 2:30 min |
| Dicafos A150 | 28,5 | | 29:56 min | 7:05 min | 4:33 min | 1:25 min | 1:50 min | 1:50 min | 2:30 min |
| HPC | 2,7 | | 35:42 min | 8:11 min | 5:11 min | 2:20 min | 2:03 min | 2:05 min | 2:57 min |
| MCC 105 | 1,8 | | | | | | | | |
| Silicon dioxide aerosil | 0,5 | | | | | | | | |
| Magnesiumstearat | 0,95 | | | | | | | | |
| Vitamin E | 30 | | 39:12 min | 13:12min | 11:30 min | 22:40 min | 23 min | 21:50 min | 24 min |
| Riboflavin | 30 | | 39:28 min | 12:56min | 11:31 min | 24 min | 23 min | 23 min | 24 min |
| MCC 200 | 20 | | 40:16 min | 13:22min | 11:40 min | 24 min | 24:30 min | 23 min | 25 min |
| Dicafos A150 | 19 | | | | | | | | |
| Magnesiumstearat | 1 | | | | | | | | |
| L-Arginin | 60 | | 15:16 min | 7:12min | 5:40 min | 9:00 min | 9:17 min | 8:33 min | 9:09 min |
| MCC 200 | 20 | | 16:10 min | 6:59min | 6:00 min | 9:57 min | 9:20 min | 8:58 min | 9:27 min |
| Dicafos A150 | 19 | | 18:34 min | 7:02min | 6:15 min | 10:12 min | 10:40 min | 9:00 min | 10:20 min |
| Magnesiumstearat | 1 | | | | | | | | |
| Dicafos A150 | 90,25 | | intact till 60 mins | less than 1 min | less than 1 min | 1:00 min | 0:56 min | 1:02 min | 0:54 min |
| MCC 200 | 7 | | | | | 1:00 min | 0:57 min | 1:02 min | 0:54 min |
| Stearic acid | 2 | | | | | 1:01 min | 0:57 min | 1:01 min | 0:55 min |
| Silicon dioxide aerosil | 0,25 | | | | | | | | |
| Magnesiumstearat | 0,5 | | | | | | | | |

Based on the results of the tablet disintegration time comparison it can be inferred that the invention has a positive influence on tablet disintegration which is comparable and in times even enhanced to the non-natural croscarmellose. Furthermore, the results show that already a small content of the premix-composition according to the present invention, of 3% w/w in the tablet formulation results in acceptable disintegration values which are improved to tablets containing no disintegrant or a known non-natural super disintegrant like croscarmellose. Due to this fact, the invention could be indicated as an excellent natural/organic super disintegrant with good properties and as potential substitute for conventional synthetic or not all natural super disintegrants.

## Claims

1. A natural disintegrant composition in powder form exclusively consisting of natural polymers which comprises from about 30% to about 90% by weight of Psyllium Husk and about 20% to about 40% by weight of Oat Fiber, about 1% to about 20% by weight of at least one further component selected from Apple Fiber, Wheat Fiber, Pea Fiber, Cellulose Powder, Agave Powder, Bamboo Fiber, and a combination thereof (all weight percentages being based on the dry weight of the disintegrant composition).

2. The natural disintegrant composition of claim 1, further comprising about 1% to about 25% by weight of at least one further component selected from guar gum, Locust bean gum, gum karaya, Aegle marmelose gum, Mangifera indica gum, and a combination thereof (all weight percentages being based on the dry weight of the disintegrant composition).

3. The natural disintegrant composition of any one of claims 1 or 2, wherein the component selected from Apple Fiber, Wheat Fiber, Pea Fiber, Cellulose Powder, Agave Powder, Bamboo Fiber, and a combination thereof is present in an amount ranging from about 1% to about 8% by dry weight of the composition.

4. The natural disintegrant composition of any one of claims 1 to 3, wherein further component selected from guar gum, Locust bean gum, gum karaya, Aegle marmelose gum, Mangifera indica gum, and a combination thereof is present in an amount ranging from about 1% to about 15% by dry weight of the composition.

5. A method for producing a natural super disintegrant composition in powder form of claim 1 exclusively consisting of natural polymers, wherein a mixture about 30% to about 90% by weight of Psyllium Husk and about 20% to about 40% by weight of Oat Fiber, and about 1% to about 25% by weight of at least one further component selected from guar gum, Locust bean gum, gum karaya, Aegle marmelose gum, Mangifera indica gum, and a combination thereof (all weight percentages being based on the dry weight of the disintegrant composition), is provided and physically blended until a homogenous mixture is attained.

6. The method of claim 5, wherein the mixture further comprises about 1% to about 20% by weight of at least one further component selected from of at least one further component selected from Apple Fiber, Wheat Fiber, Pea Fiber, Cellulose Powder, Agave Powder, Bamboo Fiber, and a combination thereof (all weight percentages being based on the dry weight of the disintegrant composition), is provided and physically blended until a homogenous mixture is attained.

7. Use of the natural disintegrant composition of any one of claims 1 to 4 for producing a solid dosage form formulation.

8. The use of claim 7, wherein the solid dosage form formulation is a tablet.

9. A solid dosage form formulation comprising the disintegrant composition of any one of claims 1 to 4 in an amount ranging from about 1% to about 10% , based on the total weight of the solid dosage form formulation on a dry weight basis.

10. The solid dosage form formulation of claim 9 comprising the disintegrant composition in an amount ranging from about 3% to about 5% w/w, based on the total weight of the tablet in the tablet formulation on a dry weight basis.

11. The solid dosage form formulation of any one of claims 9 or 10, which is in the form of a tablet.

## Patentansprüche

1. Natürliche Sprengmittelzusammensetzung in Pulverform, die ausschließlich aus natürlichen Polymeren besteht und etwa 30 bis etwa 90 Gew.-% Psyllium Husk und etwa 20 bis etwa 40 Gew.-% Haferfasern, etwa 1 bis etwa 20 Gew.-% mindestens einer weiteren Komponente, ausgewählt aus Apfelfaser, Weizenfaser, Erbsenfaser, Zellulosepulver, Agavenpulver, Bambusfaser und einer Kombination davon, umfasst (alle Gewichtsprozente beziehen sich auf das Trockengewicht der Sprengmittelzusammensetzung) .

2. Natürliche Sprengmittelzusammensetzung nach Anspruch 1, die weiterhin etwa 1 bis etwa 25 Gew.-% mindestens einer weiteren Komponente umfasst, die aus Guarkernmehl, Johannisbrotkernmehl, Karayagummi, Aegle Marmelose-Gummi, Mangifera Indica-Gummi und einer Kombination davon ausgewählt ist (alle Gewichtsprozente beziehen sich auf das Trockengewicht der Sprengmittelzusammensetzung) .

3. Natürliche Sprengmittelzusammensetzung nach einem der Ansprüche 1 oder 2, wobei die Komponente, ausgewählt aus Apfelfaser, Weizenfaser, Erbsenfaser, Zellulosepulver, Agavenpulver, Bambusfaser und einer Kombination davon, in einer Menge von etwa 1% bis etwa 8% des Trockengewichts der Zusammensetzung vorhanden ist.

4. Natürliche Sprengmittelzusammensetzung nach einem der Ansprüche 1 bis 3, wobei eine weitere Komponente, ausgewählt aus Guarkernmehl, Johannisbrotkernmehl, Karayagummi, Aegle Marmelose-Gummi, Mangifera Indica-Gummi und einer Kombination davon, in einer Menge von etwa 1% bis etwa 15% des Trockengewichts der Zusammensetzung vorhanden ist.

5. Verfahren zur Herstellung einer natürlichen Super-Sprengmittelzusammensetzung in Pulverform gemäß Anspruch 1, die ausschließlich aus natürlichen Polymeren besteht, bei dem ein Gemisch aus etwa 30 bis etwa 90 Gew.-% Psyllium Husk und etwa 20 bis etwa 40 Gew.-% Haferfasern und etwa 1 bis etwa 25 Gew.-% mindestens einer weiteren Komponente, ausgewählt aus Guarkernmehl, Johannisbrotkernmehl, Karayagummi, Aegle Marmelose-Gummi, Mangifera Indica-Gummi und einer Kombination davon (alle Gewichtsprozente beziehen sich auf das Trockengewicht der Zusammensetzung), bereitgestellt und physikalisch vermischt wird, bis ein homogenes Gemisch erreicht ist.

6. Verfahren nach Anspruch 5, wobei in dem Gemisch ferner etwa 1 bis etwa 20 Gew.-% mindestens einer weiteren Komponente, ausgewählt aus Apfelfaser, Weizenfaser, Erbsenfaser, Zellulosepulver, Agavenpulver, Bambusfaser und einer Kombination davon (alle Gewichtsprozente beziehen sich auf das Trockengewicht der Sprengmittelzusammensetzung), bereitgestellt und physikalisch vermischt wird, bis ein homogenes Gemisch erreicht ist.

7. Verwendung der natürlichen Sprengmittelzusammensetzung nach einem der Ansprüche 1 bis 4 zur Herstellung einer festen Darreichungsform.

8. Verwendung nach Anspruch 7, wobei die feste Darreichungsform eine Tablette ist.

9. Feste Darreichungsform, umfassend die Sprengmittelzusammensetzung gemäß einem der Ansprüche 1 bis 4 in einer Menge im Bereich von etwa 1% bis etwa 10%, bezogen auf das Gesamtgewicht der festen Darreichungsform auf Trockengewichtsbasis.

10. Feste Darreichungsform nach Anspruch 9, umfassend die Sprengmittelzusammensetzung in einer Menge im Bereich von etwa 3% bis etwa 5% w/w, bezogen auf das Gesamtgewicht der Tablette in der Tablettenformulierung auf Trockengewichtsbasis.

11. Feste Darreichungsform nach einem der Ansprüche 9 oder 10, die in Form einer Tablette vorliegt.

## Revendications

1. Composition à base de désintégrant naturel sous forme de poudre composée exclusivement de polymères naturels qui comprend d'environ 30 % à environ 90 % en poids d'enveloppe de psyllium et d'environ 20 % à environ 40 % en poids de fibre d'avoine, d'environ 1 % à environ 20 % en poids d'au moins un composant supplémentaire choisi parmi la fibre de pomme, la fibre de blé, la fibre de pois, la poudre de cellulose, la poudre d'agave, la fibre de bambou et une combinaison de celles-ci (tous les pourcentages en poids étant basés sur le poids sec de la composition à base de désintégrant).

2. Composition à base de désintégrant naturel selon la revendication 1, comprenant en outre d'environ 1 % à environ 25 % en poids d'au moins un composant supplémentaire choisi parmi la gomme de guar, la gomme de caroube, la gomme karaya, la gomme d'Aegle marmelose, la gomme de Mangifera indica, et une combinaison de celles-ci (tous les pourcentages en poids étant basés sur le poids sec de la composition à base de désintégrant).

3. Composition à base de désintégrant naturel selon l'une quelconque des revendications 1 ou 2, dans laquelle le composant choisi parmi la fibre de pomme, la fibre de blé, la fibre de pois, la poudre de cellulose, la poudre d'agave, la fibre de bambou et une combinaison de celles-ci est présent en une quantité allant d'environ 1 % à environ 8 % en poids sec de la composition.

4. Composition à base de désintégrant naturel selon l'une quelconque des revendications 1 à 3, dans laquelle un composant supplémentaire choisi parmi la gomme de guar, la gomme de caroube, la gomme Karaya, la gomme d'Aegle marmelose, la gomme de Mangifera indica et une combinaison de celles-ci est présent en une quantité allant d'environ 1 % à environ 15 % en poids sec de la composition.

5. Procédé de production d'une composition à base de super-désintégrant naturel sous forme de poudre selon la revendication 1 exclusivement constituée de polymères naturels, dans lequel un mélange d'environ 30 % à environ 90 % en poids d'enveloppe de psyllium et d'environ 20 % à environ 40 % en poids de fibre d'avoine, et d'environ 1 % à environ 25 % en poids d'au moins un composant supplémentaire choisi parmi la gomme de guar, la gomme de caroube, la gomme karaya, la gomme d'Aegle marmelose, la gomme de Mangifera indica, et une combinaison de celles-ci (tous les pourcentages en poids étant basés sur le poids sec de la composition à base de désintégrant), est fourni et physiquement mélangé jusqu'à l'obtention d'un mélange homogène.

6. Procédé selon la revendication 5, dans lequel le mélange comprend en outre d'environ 1 % à environ 20 % en poids d'au moins un composant supplémentaire choisi parmi la fibre de pomme, la fibre de blé, la fibre de pois, la poudre de cellulose, la poudre d'agave, la fibre de bambou et une combinaison de celles-ci (tous les pourcentages en poids étant basés sur le poids sec de la composition à base de désintégrant), et est fourni et physiquement mélangé jusqu'à l'obtention d'un mélange homogène.

7. Utilisation de la composition à base de désintégrant naturel selon l'une quelconque des revendications 1 à 4 afin de produire une formulation sous forme posologique solide.

8. Utilisation selon la revendication 7, dans laquelle la formulation sous forme posologique solide est un comprimé.

9. Formulation sous forme posologique solide comprenant la composition à base de désintégrant selon l'une quelconque des revendications 1 à 4 en une quantité allant d'environ 1 % à environ 10 %, par rapport au poids total de la formulation sous forme posologique solide sur une base de poids sec.

10. Formulation sous forme posologique solide selon la revendication 9 qui comprend la composition à base de désintégrant en une quantité allant d'environ 3 % à environ 5 % p/p, par rapport au poids total du comprimé dans la formulation de type comprimé sur une base de poids sec.

11. Formulation sous forme posologique solide selon l'une quelconque des revendications 9 ou 10, qui se présente sous la forme d'un comprimé.
